# EUROPÄISCHE PATENTSCHRIFT

(11) **EP 1 778 631 B1**
(45) Veröffentlichungstag und Bekanntmachung des Hinweises auf die Patenterteilung: **18.11.2015**
(21) Anmeldenummer: 05775924.3
(22) Anmeldetag: 14.07.2005
(51) Int. Cl.: C07C 319/18, C07C 319/20, C07C 323/22, C07C 323/60

(54) **VERFAHREN ZUR HERSTELLUNG VON 3-(METHYLTHIO)PROPANAL UND 2-HYDROXY-4-(METHYLTHIO)BUTANNITRIL**
METHOD FOR THE PRODUCTION OF 3-(METHYLTHIO)PROPANOL AND 2-HYDROXY-4-(METHYLTHIO)BUTANITRILE
PROCEDE DE PRODUCTION DE 3-(METHYLTHIO)PROPANAL ET DE 2-HYDROXY-4-(METHYLTHIO)BUTANENITRILE

(30) Priorität: 05.08.2004 DE 102004038053
(43) Veröffentlichungstag der Anmeldung: 02.05.2007
(60) Teilanmeldung: 15186588.8
(73) Patentinhaber: Evonik Degussa GmbH, 45128 Essen (DE)
(72) Erfinder: DÜBNER, Frank, 61169 Friedberg (DE); WECKBECKER, Christoph, 63584 Gründau-Lieblos (DE)
(86) Internationale Anmeldenummer: PCT/EP2005/007666
(87) Internationale Veröffentlichungsnummer: WO 2006/015684

(56) Entgegenhaltungen:
- WO-A-03/009936
- US-A- 5 705 675

## Beschreibung

Gegenstand der Erfindung betrifft ein Katalytisches Verfahren zur Herstellung von 3-(Methylthio)propanal und 2-Hydroxy-4-(methylthio)butannitril. Insbesondere beschriebt die Erfindung einen Prozess zur Darstellung von MMP und MMP-Cyanhydrin unter Verwendung neuer Additions-Katalysatoren.

### Stand der Technik

3-(Methylthio)propanal (MMP)und 2-Hydroxy-4-(methylthio)butannitril (MMP-Cyanhydrin) sind Intermediate für die Darstellung von D,L-Methionin und dem Methionin-Hydroxy-Analogen 2-Hydroxy-4-methylthiobuttersäure (MHA). Methionin ist eine essentielle Aminosäure, die u.a. als Ergänzung in Futtermitteln eingesetzt wird. MHA ist ein flüssiger Methioninersatzstoff mit geringerer biologischer Verfügbarkeit.

MMP wird durch katalysierte Addition von Methylmercaptan (Mc)an Acrolein (Ac) hergestellt. In der Regel wird flüssiges Acrolein mit Methylmercaptan in einem Reaktor zur Reaktion gebracht, in dem bereits flüssiges MMP und der Katalysator gelöst vorliegen (DT 2320544). Ebenfalls bekannt ist der Einsatz von gasförmigem Acrolein mit Methylmercaptan (FR 7520183, FR 7917827, WO 97/00858). Die Reaktion zwischen Methylmercaptan und Acrolein kann batchweise oder kontinuierlich durchgeführt werden (US 4,225,516, US 5,352,837). Als konventionelle Katalysatoren dienen organische Basen, z.B. tertiäre Amine wie Hexamethylentetramin, Trialkylamine, z.B. Triethyl- oder Triethanolamin, Benzylamine, Pyridine, z.B. 2-Fluorpyridin und 4-Dimethylaminopyridin, Picolin, Pyrazin, Imidazol und Nicotinamid, aber auch Kupfer-(II)-acetat, Quecksilbermethylmercaptid und organische Peroxide.

Das Dokument US 5,705,675 offenbart katalytische Verfahren zur Herstellung von 3-(Methylthio)propanal durch Addition von Methylmercaptan an Acrolein (Anspruch 1) und 2-Hydroxy-4-(methylthio)butanitril durch Addition von Blausäure an 3-(Methylthio)propanal (Beispiel 3).

Auch die Verwendung von Ionenaustauschern wurde erwähnt (FR 7520153). Üblicherweise wird der eigentliche Additions-Katalysator mit einem Hilfskatalysator, einer organischen Säure, z.B. Essigsäure, Zitronensäure oder Ameisensäure oder einer Mineralsäure, z.B. Schwefel- oder Phosphorsäure kombiniert, um zum einen die Polymerisation von Acrolein, also die Bildung von unerwünschten Nebenprodukten, zu inhibieren und zum anderen die generelle Ausbeute durch Konditionierung der zugesetzten Base zu erhöhen. Der Katalysator wird nicht zurückgewonnen und geht während der Aufarbeitung verloren.

Typische Katalysatorkonzentrationen liegen bei 0,001 bis 0,005 Mol-% bezogen auf Methylmercaptan. Die notwendige Menge an Säure, typischerweise Essigsäure, liegt zwischen 0,5 und 50 Mol-%. Zur Vereinfachung der MMP-Herstellverfahren können Katalysator und Säure in einem Prämix vorgemischt und als Lösung zudosiert werden. Die Konzentration an Katalysator-Prämix im flüssigen MMP-Reaktionsmedium beträgt üblicherweise 0,2 bis 0,75 Gew. Nach beendeter Reaktion wird das MMP von den Hilfs- und Nebenprodukten durch Destillation getrennt. Während der destillativen Aufreinigung des so hergestellten Additionsproduktes geht der Katalysator-Prämix verloren und muss je nach Siedepunkt über den Destillationssumpf oder das Abgas entsorgt werden. Grundsätzlich können Teile des Katalysators oder der zugesetzten Säure während der Destillation über Kopf gehen und das gewünschte Rein-MMP verunreinigen.

Aus MMP lässt sich durch Reaktion mit Cyanwasserstoff (Blausäure) unter Verwendung geeigneter Katalysatoren das MMP-Cyanhydrin 2-Hydroxy-4-(methylthio)butannitril herstellen. Geeignete Katalysatoren sind oben genannte Basen, die auch die Addition von Methylmercaptan an Acrolein katalysieren, z.B. Pyridin oder Triethylamin. Durch Hydrolyse von MMP-Cyanhydrin mit z.B. Mineralsäuren erhält man MHA. Methionin entsteht durch Reaktion von MMP-Cyanhydrin mit Ammoniumhydrogencarbonat unter Bildung von Hydantoin, das mit einer Base, z.B. Kaliumcarbonat oder Natriumhydroxid verseift werden kann. Die Freisetzung des Methionins geschieht mit Kohlendioxid oder Schwefelsäure.

### Aufgabe der Erfindung

Es war Aufgabe dieser Erfindung, ein katalytisches Verfahren bereitzustellen, dass sowohl die Addition von Methylmercaptan an Acrolein, als auch die weitere Umsetzung des Reaktionsproduktes zu MMP-Cyanhydrin ermöglicht, ohne dabei den Katalysator oder eine zusätzliche Säure zu verbrauchen.

### Beschreibung der Erfindung

Die Erfindung ist ein Verfahren zur Herstellung von 3-(Methylthio)propanal, bei dem ein neuartiges Katalysatorsystem die Addition von Methylmercaptan an Acrolein ermöglicht. Weiterhin ist beschrieben ein nicht erfindungsgemäßes Verfahren, bei dem dieses neuartige Katalysatorsystem die Reaktion zwischen MMP und Blausäure zur Darstellung von MMP-Cyanhydrin katalysiert.

Insbesondere ist die Erfindung ein Verfahren zur kontinuierlichen Herstellung von MMP unter Verwendung heterogener Katalysatoren, die im Reaktionsmedium unlöslich sind und daher nicht verbraucht werden. Die Zugabe von Hilfskatalysatoren, wie organischen Säuren, z.B. Essigsäure, ist nicht notwendig.

Die neuen Katalysatoren haben die allgemeine Formel (I) 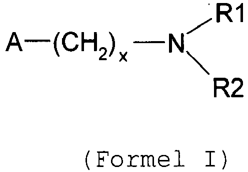 wobei R₁ und R₂ Wasserstoff, Alkyl mit Kettenlängen zwischen C₁ und C₁₂, Aryl oder Heteroaryl sind. R₁ kann verschieden von R₂ sein. X ist eine Zahl zwischen 0 und 6. A ist ein natürliches oder synthetisches Harz, z.B. Polystyrol. Insbesondere werden die Polymer-gebundenen Basen aus der Reihe der homologen Dialkylaminoalkylpolystyrole und Dialkylaminomakroretikulären Harze, besonders aus Dimethylaminoethylpolystyrol, Diethylaminoethylpolystyrol, Dimethylaminomethylpolystyrol, Diethylaminomethylmakroretikuläres Harz und vor allen Dingen Diethylaminomethylpolystyrol gewählt. Die genannten Basen finden teilweise bereits in anderen Bereichen der Festphasenchemie Anwendung (WO 03/009936, US 4,440,676) und einige sind kommerziell erhältlich. Weiterhin ist die Synthese spezieller Derivate einfach aus beschriebenen Harzen, z.B. Merrifield-Harz, möglich.

Da die Katalysatoren weder im Reaktionsprodukt, noch in einem der beteiligten Reaktionspartner löslich sind, ist eine für die Reaktion beliebige Katalysatormenge, die zu ausreichenden Umsätzen und Selektivitäten führt, einsetzbar. Zur Erleichterung der Reaktionsführung, d.h. Dosierung der Reaktionspartner, Transport der Edukte an die aktiven Zentren des Katalysators und Abführung der Reaktionswärme, sollte ein Reaktionsmedium vorhanden sein, in dem der Katalysator gut quellbar ist. Gut quellbar ist ein Harz in einem Lösungsmittel dann, wenn es die gleiche bis fünffache Eigenmasse an Lösungsmittel aufnehmen kann. Das Harz vergrößert dabei unter Aufnahme des Lösungsmittels sein Volumen. Vorzugsweise kann MMP bzw. nicht erfindungsgemäß MMP-Cyanhydrin selbst als Matrix für die Reaktion dienen. Aber auch alle gängigen Lösungsmittel, in denen die Reaktionspartner, nicht aber der Katalysator, zumindest teilweise löslich sind, z.B. Kohlenwasserstoffe, halogenierte Kohlenwasserstoffe oder Ether sind einsetzbar. Reaktive Lösungsmittel wie Wasser, Alkohole und Ketone, die mit den Edukten oder den Produkten unerwünschte Nebenprodukte bilden können, sind weniger geeignet und nur bedingt einsetzbar. Bezogen auf die aktiven Zentren bietet sich in Batchversuchen zur Darstellung von MMP ein molares Verhältnis von Katalysator zu Acrolein von 0,001 bis zu 0,02, vorzugsweise von 0,001 bis zu 0,01, besonders bevorzugt von 0,001 bis zu 0,005 an. In kontinuierlichen Umsetzungen, bei denen die Reaktionspartner und das Reaktionsmedium kontinuierlich am Katalysator vorbeiströmen, beträgt das Verhältnis aus Acroleinmasse zu Katalysatormasse pro Stunde (LHSV-Wert, m/m*h) zwischen 0,1 und 100, vorzugsweise zwischen 1 und 50, besonders bevorzugt zwischen 5 und 50.

Das Verhältnis zwischen dem Reaktionsmedium zu Acrolein wird in Batchversuchen zwischen 0,1 und 2 gewählt. In kontinuierlichen Verfahren sollte das Massenstromverhältnis von Reaktionsmedium zu Acrolein zwischen 0,5 und 20 liegen. Zur Erzielung einer guten Umsetzung der Reaktanten und zur Erreichung geringen Abbaus, werden die Mengen der eingesetzten Edukte so geregelt, dass ein leichter Überschuss an Methylmercaptan im Reaktionsgemisch aufrecht erhalten wird. Der Überschuss sollte auf molarer Basis zwischen 0,01 und 1%, vorzugsweise zwischen 0,05 und 0,2% liegen. Ein Überschuss an Acrolein führt zu vermehrter Bildung hochsiedender Rückstände und ist daher nicht wünschenswert.

Bei Durchführung der Reaktion ist Druck kein kritischer Faktor. Er kann innerhalb weiter Grenzen schwanken. Da ein zu hoher Druck jedoch durch Kompression des Katalysatorbetts dessen Aktivität vermindern kann, sollten Drücke oberhalb 10 bar vermieden werden. Es ist bevorzugt die Reaktion bei Atmosphärendruck durchzuführen.

Die Reaktionstemperatur kann je nach Druck und Reaktionsmedium zwischen -20 und 100°C gewählt werden. Bei Atmosphärendruck und MMP als Matrix bieten sich Temperaturen zwischen 0 und 60°C an, insbesondere Temperaturen zwischen 30 und 50°C. Oberhalb dieser Temperaturen sinkt die Selektivität bzgl. MMP-Bildung, unterhalb von 0°C dagegen ist die Reaktionsgeschwindigkeit zu gering um wirtschaftlich Vorteile bringen zu können.

Bei der batchweisen Produktion von MMP wird im Reaktor ein Startinhalt an MMP bzw. Reaktionsmedium vorgelegt. Der Katalysator wird in diesem Reaktionsmedium suspendiert. Es ist notwendig, den Katalysator vor Beginn der Reaktion, d.h. bei erstmaligem Einsatz, im Reaktionsmedium quellen zu lassen, um eine bestmögliche Zugänglichkeit der aktiven Zentren zu ermöglichen. Anschließend werden die Startmaterialien Methylmercaptan und Acrolein zeitgleich eingeleitet. Die Reaktionswärme wird durch geeignete Ein- oder Umbauten abgeführt.

Es ist vorteilhaft, zunächst ca. 10% des Methylmercaptans vorzulegen, und erst dann mit der Einleitung von Acrolein und des restlichen Methylmercaptans fortzufahren. Methylmercaptan löst sich mit Wärmetönung unter Ausbildung eines Hemithioacetals in MMP. Auf diese Weise wird ein kontinuierlicher Überschuss an Methylmercaptan während der Reaktion gewährleistet. Ein Überschuss an Methylmercaptan führt zu höheren Selektivitäten und damit zur Minimierung hochsiedender Nebenprodukte. Bei ausreichender Vorlage an MMP, ist es möglich das komplette Methylmercaptan vorzulegen, und erst anschließend Acrolein zuzugeben.

Nach beendeter Reaktion wird das Produkt vom Katalysator abfiltriert und kann gegebenenfalls weiter aufgereinigt werden. Ist das Reaktionsprodukt MMP nicht im Reaktionsmedium löslich, kann dieses durch Phasentrennung vom Reaktionsmedium separiert werden. Der Katalysator und das Reaktionsmedium können direkt und ohne weiteres Quellen in Folgereaktionen wieder eingesetzt werden. Bei Verwendung von MMP als Reaktionsmedium, ist es von Vorteil, nur einen Teil des gebildeten MMPs auszuschleusen, und einen entsprechenden Betriebsinhalt mit dem Katalysator für folgende Chargen zurück zu halten.

Soll das Roh-MMP weiter zu MMP-Cyanhydrin umgesetzt werden, wie hier beschrieben, gelingt dies einfach durch Zudosierung von äquimolaren Mengen Blausäure in das Reaktionsmedium mit erfindungsgemäßem Katalysator. In der batchweisen Produktion muss dazu die vollständige Umsetzung des Acroleins mit Methylmercaptan abgeschlossen sein. Eine Aufreinigung des Roh-MMPs ist nicht notwendig.

In der kontinuierlichen Produktion kann die Dosierung der Blausäure an einer Stelle des Katalysatorbetts erfolgen, an der der Umsatz zum MMP komplett ist. Möglich ist auch eine nachgeschaltete Reaktionsschleife mit separatem Katalysatorbett. Zur Vermeidung unnötiger Nebenprodukte sollte die Reaktion mit einem Überschuss an Blausäure gefahren werden. Der molare Überschuss an Blausäure bezogen auf MMP sollte zwischen 0,01 und 10%, vorzugsweise zwischen 0,05 und 1% liegen. Die Reaktionstemperatur sollte zwischen 0 und 100°C, vorzugsweise zwischen 20 und 70°C liegen. Es kann sowohl verflüssigte, als auch gasförmige Blausäure eingesetzt werden.

Kurze Beschreibung der Figuren
Figur 1 stellt einen schematischen Aufbau einer kontinuierlichen Produktion von MMP mit dem erfindungsgemäßen heterogenen, Festphasenkatalysator in einem Festbettreaktor dar.
Figur 2 stellt einen schematischen Aufbau einer kontinuierlichen Produktion von MMP mit dem erfindungsgemäßen heterogenen, Festphasenkatalysator in einem Festbettreaktor dar, erweitert für die nicht erfindungsgemäße Umsetzung von MMP zu MMP-Cyanhydrin mit einer Blausäuredosierung bzw. gegebenenfalls um eine separate Cyanhydrin-Reaktionsschleife.

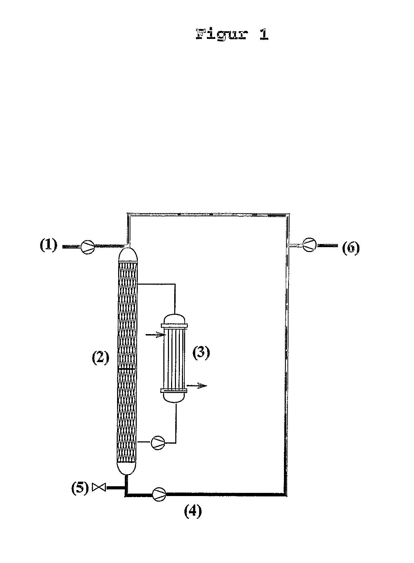

In Figur 1 ist der zentrale Apparat ein Festbettreaktor mit Katalysatorfüllung und Kreislaufpumpe. An den Kreislauf, der auch einen Wärmetauscher beinhaltet, sind Dosiereinrichtungen zum Einleiten von Methylmercaptan und Acrolein, und eine Ausschleusung zur Entnahme des entstandenen MMP-Reaktionsgemisches vorhanden. Ist das Reaktionsmedium verschieden von MMP, muss aufgrund der kontinuierlichen Ausschleusung eine entsprechende Menge des entnommenen Lösungsmittels in die Reaktionsschleife nachdosiert werden. Vorzugsweise befindet sich die Methylmercaptan-Dosierung vor der Acroleinzugabe. Dies gewährleistet einen zeitlichen Überschuss an Methylmercaptan zu Beginn der Reaktion. Dennoch kann es von Vorteil sein einen entgegen der nötigen Stöchiometrie im Mittel geringen Überschuss an Methylmercaptan zuzugeben. Der Überschuss kann zwischen 0,01 und 1% liegen. Höhere Mengen führen zu keiner Verbesserung der Acroleinausbeute.

Die Verweilzeit der Reaktanten am Katalysator wird über das Katalysatorvolumen und den Fluss bestimmt. Vorzugsweise beträgt das Verhältnis aus Acroleinmasse zu Katalysatormasse pro Stunde (LHSV-Wert, m/m*h) zwischen 0,1 und 100, bevorzugt zwischen 1 und 50, besonders bevorzugt zwischen 5 und 50. Das Massenverhältnis Reaktionsmedium zu Acrolein sollte zwischen 1 und 20 liegen. Die Wärmeabfuhr am Katalysator wird so geregelt dass eine Temperatur zwischen 30 und 50°C erreicht wird. Die Reaktion wird drucklos gefahren. Erhöhte Drücke sind möglich, haben aber keinen Einfluss auf den Umsatz.

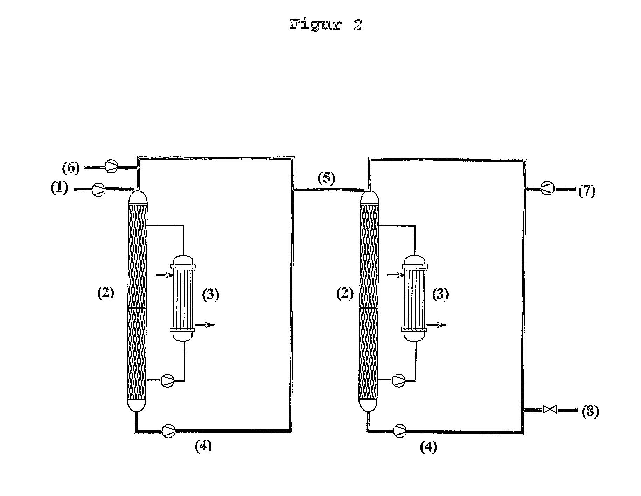

Soll das erzeugte MMP weiter zu MMP-Cyanhydrin umgesetzt werden, erweitert sich der Aufbau um eine Blausäuredosierung bzw. gegebenenfalls um eine separate Cyanhydrin-Reaktionsschleife (nicht erfindungsgemäß) wie in Figur 2 dargestellt.

Das erfindungsgemäße Verfahren hat den Vorteil, keinen Katalysator oder Katalysatorhilfsstoff zu verbrauchen. Dies ist kosteneffizient und nachhaltig. Weiterhin wird das Reaktionsprodukt MMP bzw. MMP-Cyanhydrin nicht durch Katalysatoren oder Katalysatorenhilfsstoffe verunreinigt. Das erleichtert zum einen die Aufarbeitung der Produkte und minimiert zum anderen unerwünschte Nebenreaktionen in Folgestufen, die durch Reste der Katalysatoren hervorgerufen werden.

Die vorliegende Erfindung wird im folgenden anhand von Ausführungsbeispielen näher erläutert. Diese dienen nur zur Veranschaulichung der Erfindung und sind in keinem Fall als Limitierung in Art und Umfang dieser zu sehen.

### Beispiel 1

### 1.1 Darstellung von Dimethylaminomethylpolystyrolharz

In einen handelsüblichen Laborautoklav werden 5 g (4,5 mmol) Merrifieldharz [CAS 55844-94-5] (0,9 mmol Cl/g), 6,9 g (50 mmol) Triethylamin und 200 ml Dimethylamin-Lösung (400 mmol, 2 M in Tetrahydrofuran, THF)gegeben. Die Mischung wird für fünf Stunden auf 85°C erwärmt. Nach Abkühlung und Entspannung wird über eine Glasfritte abgesaugt und der Filterkuchen erst mit Wasser Chlorid-frei gewaschen und anschließend noch mit 200 ml THF nachgewaschen. Das bei 60°C getrocknete Harz kann nach Quellung direkt in Folgeversuchen eingesetzt werden. Man erhält 4,8 g Produkt, das nach NMR zu >90% aus dem Dimethylbenzylamin -funktionalisierten Harz besteht. Neben nicht reagiertem Benzylchlorid ist Benzylalkohol eine Nebenfunktionalität.

### 1.2 Batchweise Darstellung von 3-(Methylthio)propanal

In einem Reaktionskolben mit Tropftrichter und Gaseinleitung werden 0,5 g Dimethylaminomethylpolystyrolharz (Aktivität ca. 18 mmol/l MMP) in 25 ml destilliertem Rein-MMP als Vorlage dispergiert. Man lässt das Harz 1h quellen. Bei 0°C leitet man anschließend innerhalb von 10 min 10 g (208 mmol) Methylmercaptan ein, welches sofort unter Bildung eines MMP-Halbmercaptals in Lösung geht. Nach beendeter Einleitung werden 11,5 g (205 mmol) Acrolein zugetropft und weiter bei 0°C gerührt. Nach zwei Stunden wird der Katalysator abfiltriert und das MMP analysiert. Man erhält einen Acroleinumsatz von 98% und eine Ausbeute von 95%. Der Rückstand im Sumpf bei Destillation des Roh-MMPs beträgt 0,19%.

Der abfiltrierte Katalysator kann direkt ohne weiteres Quellen in Folgeversuchen eingesetzt werden. Ein zehnfacher Einsatz zeigt keinen Aktivitätsverlust.

### 1.3 Batchweise Darstellung von 2-Hydroxy-4-(methylthio)-butannitril (nicht erfindungsgemäß)

Zur weiteren Umsetzung mit Blausäure, wird der Katalysator nicht abfiltriert, sondern zu der MMP-Reaktionsmischung unter Kühlung bei ca. 35°C innerhalb von 30 min. Blausäure (12,6 g, 466 mmol, 1,05 Äq.) zugetropft. Nach beendeter Reaktion wird vom Katalysator abfiltriert. Man erhält 62 g MMP-Cyanhydrin mit einer Reinheit von >98%. Die mehrfache Wiederverwendung des Katalysators führt zu keinem Aktivitätsverlust.

### 1.4 Kontinuierliche Darstellung von 3-(Methylthio)propanal

In ein Reaktionsrohr werden 4 mmol zuvor in MMP gequollenes Dimethylaminomethylpolystyrolharz (entspr. 4,4 g trocken) gefüllt. Der Rohrreaktor wird in eine mit Rein-MMP beschickte Umlaufschleife mit Pumpe eingebunden. Das Fassungsvermögen in der Schleife beträgt ca. 5 ml. Eine weitere Pumpe erlaubt das Einbringen von Acrolein vor das Reaktionsrohr. Weiterhin ermöglicht ein Ventil das Einleiten von flüssigem oder gasförmigen Methylmercaptan in den Strom. Ein Begleitwärmetauscher dient zur Temperierung der Reaktionsschleife auf 50°C. Zum Starten der Reaktion werden 0,25 g Acrolein / min und 0,21 g Methylmercaptan / min zudosiert. Die der Reaktionsschleife zugeführte Volumenmenge wird drucklos an einer Ausschleusstelle entnommen. Das Volumenverhältnis Zulauf zu Kreislauf beträgt 1/5. Die Reaktion ist nach ca. 30 min im stationären Zustand. Die Analyse einer repräsentativen Roh-MMP-Probe ergibt Acroleinumsätze > 99% und MMP-Reinheiten von ca. 93%.

Die der Reaktionsschleife entnommene Menge an MMP wird in einer zweiten Reaktionsschleife zum MMP-Cyanhydrin umgesetzt (nicht erfindungsgemäß). Aufbau und Fahrweise entsprechen obiger Darstellung zur Reaktion von Acrolein mit Methylmercaptan. Die Katalysatormenge beträgt 4 mmol, die Zulaufmenge an HCN 0,12 g / min. Die der Reaktionsschleife zugeführte Volumenmenge wird drucklos an einer Ausschleusstelle entnommen. Das Volumenverhältnis Zulauf zu Kreislauf beträgt 1/5, die Temperatur wird auf 40°C gehalten. Die Reaktion ist nach ca. 30 min im stationären Zustand. Die Analyse einer repräsentativen MMP-Cyanhydrin-Probe ergibt MMP-Umsätze > 99% und Reinheiten von ca. 92%.

### Beispiel 2

### 2.1 Darstellung von Diethylaminomethylpolystyrolharz

In einem Rührkolben mit Rückflusskühler werden 30 g (27 mmol) Merrifieldharz [CAS 55844-94-5] (0,9 mmol Cl/g), 30,4 g (300 mmol) Triethylamin und 87,8 g (1,20 mol) Diethylamin in 420 ml Methylisobutylketon suspendiert. Die Mischung wird für 6 Stunden am Rückfluss gehalten. Nach Abkühlung auf Raumtemperatur wird über eine Glasfritte abgesaugt und der Filterkuchen mit Wasser Chlorid-frei gewaschen. Das bei 60°C getrocknete Harz kann nach Quellung direkt in Folgeversuchen eingesetzt werden. Man erhält 32 g Produkt, dass nach NMR zu >90% aus dem Diethylbenzylaminfunktionalisierten Harz besteht. Neben nicht reagiertem Benzylchlorid ist Benzylalkohol eine Nebenfunktionalität.

### 2.2 Batchweise Darstellung von 3-(Methylthio)propanal

Entsprechend der Ausführung aus Beispiel 1 werden 0,5 g Diethylaminomethylpolystyrolharz mit Acrolein und Methylmercaptan umgesetzt.

Nach 2 Stunden erhält man einen Acroleinumsatz von >99% und eine Ausbeute von >96%. Der Rückstand im Sumpf bei Destillation des Roh-MMPs beträgt 0,15%.

Die weitere Umsetzung zum MMP-Cyanhydrin (nicht erfindungsgemäß) liefert dieses bei Umsätzen >99% in einer Reinheit von >95%.

Der abfiltrierte Katalysator kann direkt ohne weiteres Quellen in Folgeversuchen eingesetzt werden. Ein zehnfacher Einsatz zeigt keinen Aktivitätsverlust.

### 2.3 Kontinuierliche Darstellung von 3-(Methylthio)propanal

Entsprechend der Ausführung aus Beispiel 1 werden 4,4 g (ca. 4 mmol) Diethylaminomethylpolystyrolharz mit Acrolein und Methylmercaptan umgesetzt.

Die Analyse einer repräsentativen Roh-MMP-Probe ergibt Acroleinumsätze > 99% und MMP-Reinheiten von ca. 94%, während die Analyse einer repräsentativen MMP-Cyanhydrin-Probe nach Zugabe von Blausäure (nicht erfindungsgemäß) MMP-Umsätze > 99% und Reinheiten von ca. 93% ergibt.

### Erklärungen zu den Figuren:

(1) Eintrag von Acrolein
(2) Katalysator
(3) Wärmeaustauscher
(4) Kreislaufpumpe
(5) Austrag von MMP
(6) Eintrag von Methylmercaptan
(7) Eintrag von Blausäure (nicht erfindungsgemäß)
(8) Austrag von MMP-Cyanhydrin (nicht erfindungsgemäß)

## Patentansprüche

1. Verfahren zur Herstellung von 3-(Methylthio)propanal durch Addition von Methylmercaptan an Acrolein, in Gegenwart von a) einem heterogenen Katalysator, und b) einem Reaktionsmedium, **dadurch gekennzeichnet, dass** der heterogene Katalysator in dem Reaktionsmedium nicht löslich ist.

2. Verfahren gemäß Anspruch 1, **dadurch gekennzeichnet, dass** der heterogene Katalysator die allgemeine Formel 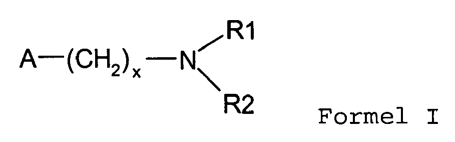 hat, wobei
R₁ und R₂ Wasserstoff, Alkyl mit Kettenlängen zwischen C₁ und C₁₂, Aryl oder Heteroaryl sind;
R₁ kann verschieden von R₂ sein;
X ist eine Zahl zwischen 0 und 6; und
A ist ein natürliches oder synthetisches Harz.

3. Verfahren gemäß Anspruch 2, **dadurch gekennzeichnet, dass** A in der Formel I ein Polystyrol ist.

4. Verfahren gemäß Anspruch 2, **dadurch gekennzeichnet, dass** der Katalysator gemäß Formel I eine Polymergebundene Base ausgewählt aus der Gruppe der homologen Dialkylaminoalkylpolystyrole oder Dialkylaminomakroretikulären Harze ist.

5. Verfahren gemäß Anspruch 4, **dadurch gekennzeichnet dass** der Katalysator gemäß Formel I Diethylaminoethylpolystyrol, Diethylaminomethylpolystyrol, Dimethylaminomethylpolystyrol, Diethylaminomethylmakroretikuläres Harz oder Dimethylaminoethylpolystyrol ist.

6. Verfahren gemäß Anspruch 1, **dadurch gekennzeichnet, dass** das Reaktionsmedium ein Kohlenwasserstoff, ein halogenierter Kohlenwasserstoff, ein Ether, 3-(Methylthio)propanal oder 2-Hydroxy-4-(methylthio)-butannitril ist.

7. Verfahren gemäß Anspruch 6, **dadurch gekennzeichnet, dass** das Reaktionsmedium 3-(Methylthio)propanal ist.

8. Verfahren gemäß Anspruch 1, **dadurch gekennzeichnet, dass** es bei Temperaturen von -20°C bis 100°C durchgeführt wird.

9. Verfahren gemäß Anspruch 8, **dadurch gekennzeichnet, dass** die Temperatur zwischen 0°C und 60°C beträgt.

10. Verfahren gemäß Anspruch 8 oder 9, **dadurch gekennzeichnet, dass** die Temperatur zwischen 30°C und 50°C beträgt.

11. Verfahren gemäß Anspruch 1, **dadurch gekennzeichnet, dass** bezogen auf die aktiven Zentren das molare Verhältnis von Katalysator zu Acrolein von 0,001 bis zu 0,02 ist.

12. Verfahren gemäß Anspruch 11, **dadurch gekennzeichnet, dass** das molare Verhältnis von Katalysator zu Acrolein, von 0,001 bis zu 0,01 ist.

13. Verfahren gemäß Anspruch 12, **dadurch gekennzeichnet, dass** das molare Verhältnis von Katalysator zu Acrolein von 0,001 bis zu 0,005 ist.

14. Verfahren gemäß Anspruch 1, **dadurch gekennzeichnet, dass** der molare Überschuss an Methylmercaptan bezogen auf Acrolein zwischen 0,01 und 1%, vorzugsweise zwischen 0,05 und 0,2% ist.

15. Verfahren gemäß Anspruch 1, **dadurch gekennzeichnet, dass** das Verfahren ein Batch-Verfahren ist.

16. Verfahren gemäß Anspruch 15, **dadurch gekennzeichnet, dass** das Verhältnis von Reaktionsmedium zu Acrolein zwischen 0,1 und 2 ist.

17. Verfahren gemäß Anspruch 1, **dadurch gekennzeichnet, dass** das Verfahren kontinuierlich ist.

18. Verfahren gemäß Anspruch 17, **dadurch gekennzeichnet, dass** das Massenstromverhältnis von Reaktionsmedium zu Acrolein zwischen 0,5 und 20 ist.

## Claims

1. Method for preparing 3-(methylthio)propanal by addition of methylmercaptan to acrolein in the presence of a) a heterogeneous catalyst, and b) a reaction medium, **characterized in that** the heterogeneous catalyst is not soluble in the reaction medium.

2. Method according to Claim 1, **characterized in that** the heterogeneous catalyst has the general formula 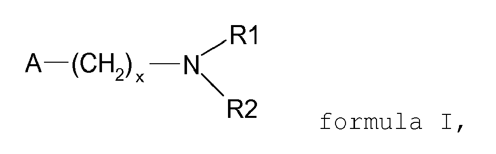 where
R₁ and R₂ are hydrogen, alkyl having chain lengths between C₁ and C₁₂, aryl or heteroaryl;
R₁ can be different from R₂;
X is a number between 0 and 6; and
A is a natural or synthetic resin.

3. Method according to Claim 2, **characterized in that** A in the formula I is a polystyrene.

4. Method according to Claim 2, **characterized in that** the catalyst according to formula I is a polymer-bound base selected from the group consisting of the homologous dialkylaminoalkylpolystyrenes or dialkylaminomacroreticular resins.

5. Method according to Claim 4, **characterized in that** the catalyst according to formula I is diethylaminoethylpolystyrene, diethylaminomethylpolystyrene, dimethylaminomethylpolystyrene, diethylaminomethylmacroreticular resin or dimethylaminoethylpolystyrene.

6. Method according to Claim 1, **characterized in that** the reaction medium is a hydrocarbon, a halogenated hydrocarbon, an ether, 3-(methylthio)propanal or 2-hydroxy-4-(methylthio)butanenitrile.

7. Method according to Claim 6, **characterized in that** the reaction medium is 3-(methylthio)propanal.

8. Method according to Claim 1, **characterized in that** it is carried out at temperatures of -20°C to 100°C.

9. Method according to Claim 8, **characterized in that** the temperature is between 0°C and 60°C.

10. Method according to Claim 8 or 9, **characterized in that** the temperature is between 30°C and 50°C.

11. Method according to Claim 1, **characterized in that**, based on the active centres, the molar ratio of catalyst to acrolein is from 0.001 to 0.02.

12. Method according to Claim 11, **characterized in that** the molar ratio of catalyst to acrolein is from 0.001 to 0.01.

13. Method according to Claim 12, **characterized in that** the molar ratio of catalyst to acrolein is from 0.001 to 0.005.

14. Method according to Claim 1, **characterized in that** the molar excess of methylmercaptan based on acrolein is between 0.01 and 1%, preferably between 0.05 and 0.2%.

15. Method according to Claim 1, **characterized in that** the method is a batch method.

16. Method according to Claim 15, **characterized in that** the ratio of reaction medium to acrolein is between 0.1 and 2.

17. Method according to Claim 1, **characterized in that** the method is continuous.

18. Method according to Claim 17, **characterized in that** the ratio of flow rates by mass of reaction medium to acrolein is between 0.5 and 20.

## Revendications

1. Procédé de fabrication de 3-(méthylthio)propanal par addition de méthylmercaptan sur de l'acroléine, en présence de a) un catalyseur hétérogène et b) un milieu réactionnel, **caractérisé en ce que** le catalyseur hétérogène n'est pas soluble dans le milieu réactionnel.

2. Procédé selon la revendication 1, **caractérisé en ce que** le catalyseur hétérogène a la formule générale 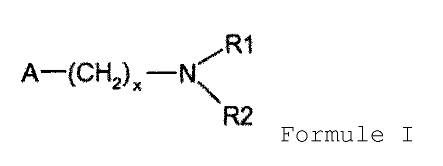 dans laquelle
R₁ et R₂ représentent hydrogène, alkyle de longueur de chaîne comprise entre C₁ et C₁₂, aryle ou hétéroaryle ;
R₁ peut être différent de R₂ ;
X représente un nombre compris entre 0 et 6 ; et
A représente une résine naturelle ou synthétique.

3. Procédé selon la revendication 2, **caractérisé en ce que**, dans la formule I, A représente un polystyrène.

4. Procédé selon la revendication 2, **caractérisé en ce que** le catalyseur de formule I est une base reliée à un polymère choisie dans le groupe des dialkylaminoalkylpolystyrènes homologues ou des résines dialkylamino macroréticulaires.

5. Procédé selon la revendication 4, **caractérisé en ce que** le catalyseur de formule I est le diéthylaminoéthylpolystyrène, le diéthylaminométhylpolystyrène, le diméthylaminométhylpolystyrène, une résine diéthylaminométhyle macroréticulaire ou le diméthylaminoéthylpolystyrène.

6. Procédé selon la revendication 1, **caractérisé en ce que** le milieu réactionnel est un hydrocarbure, un hydrocarbure halogéné, un éther, le 3-(méthylthio)propanal ou le 2-hydroxy-4-(méthylthio)-butane-nitrile.

7. Procédé selon la revendication 6, **caractérisé en ce que** le milieu réactionnel est le 3-(méthylthio)propanal.

8. Procédé selon la revendication 1, **caractérisé en ce qu'**il est réalisé à des températures de -20 °C à 100 °C.

9. Procédé selon la revendication 8, **caractérisé en ce que** la température est comprise entre 0 °C et 60 °C.

10. Procédé selon la revendication 8 ou 9, **caractérisé en ce que** la température est comprise entre 30 °C et 50 °C.

11. Procédé selon la revendication 1, **caractérisé en ce que**, par rapport aux centres actifs, le rapport molaire entre le catalyseur et l'acroléine est de 0,001 à 0,02.

12. Procédé selon la revendication 11, **caractérisé en ce que** le rapport molaire entre le catalyseur et l'acroléine est de 0,001 à 0,01.

13. Procédé selon la revendication 12, **caractérisé en ce que** le rapport molaire entre le catalyseur et l'acroléine est de 0,001 à 0,005.

14. Procédé selon la revendication 1, **caractérisé en ce que** l'excès molaire de méthylmercaptan par rapport à l'acroléine est compris entre 0,01 et 1 %, de préférence ente 0,05 et 0,2 %.

15. Procédé selon la revendication 1, **caractérisé en ce que** le procédé est un procédé discontinu.

16. Procédé selon la revendication 15, **caractérisé en ce que** le rapport entre le milieu réactionnel et l'acroléine est compris entre 0,1 et 2.

17. Procédé selon la revendication 1, **caractérisé en ce que** le procédé est continu.

18. Procédé selon la revendication 17, **caractérisé en ce que** le rapport entre les courants massiques du milieu réactionnel et de l'acroléine est compris entre 0,5 et 20.
